# EUROPEAN PATENT SPECIFICATION

(11) **EP 1 303 597 B1**
(45) Date of publication and mention of the grant of the patent: **31.12.2008**
(21) Application number: 01940942.4
(22) Date of filing: 30.05.2001
(51) Int. Cl.: C12N 15/11, C12N 9/00

(54) **METHOD OF RNA CLEAVAGE**
VERFAHREN ZUR RNA-SPALTUNG
METHODE DE CLIVAGE D'ARN

(30) Priority: 31.05.2000 US 208432 P
(43) Date of publication of application: 23.04.2003
(73) Proprietor: Istituto di Biologia Cellulare, CNR, 00016 Monterotondo Scalo (IT)
(72) Inventor: TOCCHINI-VALENTINI, Glauco, P., I-00191 Roma (IT); DEIDDA, Giancarlo, I-00156 Roma (IT); ROSSI, Nicoletta, I-00155 Roma (IT); BALDI, Maria, Irene, I-00199 Roma (IT); FRUSCOLONI, Paolo, I-00141 Roma (IT)
(74) Representative: Duckworth, Timothy John
(86) International application number: PCT/IB2001/001189
(87) International publication number: WO 2001/092463

(56) References cited:
- WO-A-95/32283
- FABBRI STEFANIA ET AL: "Conservation of substrate recognition mechanisms by tRNA splicing endonucleases." SCIENCE (WASHINGTON D C), vol. 280, no. 5361, 10 April 1998 (1998-04-10), pages 284-286, XP002216303 ISSN: 0036-8075 cited in the application
- NASHIMOTO MASAYUKI ET AL: "RNA heptamers that direct RNA cleavage by mammalian tRNA 3' processing endoribonuclease." NUCLEIC ACIDS RESEARCH, vol. 26, no. 11, 1 June 1998 (1998-06-01), pages 2565-2571, XP002216304 ISSN: 0305-1048
- LI HONG ET AL: "Crystal structure and evolution of a transfer RNA splicing enzyme." SCIENCE (WASHINGTON D C), vol. 280, no. 5361, 10 April 1998 (1998-04-10), pages 279-284, XP002216305 ISSN: 0036-8075
- DIENER JOHN L ET AL: "Solution structure of a substrate for the archaeal pre-tRNA splicing endonucleases: The bulge-helix-bulge motif." MOLECULAR CELL, vol. 1, no. 6, May 1998 (1998-05), pages 883-894, XP002216306 ISSN: 1097-2765 cited in the application
- FRUSCOLONI PAOLO ET AL: "Cleavage of non-tRNA substrates by eukaryal tRNA splicing endonucleases." EMBO REPORTS, vol. 2, no. 3, March 2001 (2001-03), pages 217-221, XP002216307 ISSN: 1469-221X

## Description

### BACKGROUND OF THE INVENTION

Accuracy in tRNA splicing is essential for the formation of functional tRNAs and, hence, for gene expression. In Bacteria, tRNA introns are self-splicing group I introns and the splicing mechanism is autocatalytic. In Eukarya, tRNA introns are small and invariably interrupt the anticodon loop one base 3' to the anticodon. In Archaea, the introns are also small and often reside in the same location as eukaryal tRNA introns.

In both Eukaryotes and Archaea, the specificity for recognition of the pre-tRNA resides in the endonucleases. These enzymes remove the intron by making two independent endonucleotytic cleavages. The archaeal enzyme acts without any reference to the mature domain (mature-domain independent mode, MDI) but instead recognizes a structure, the bulge-helix-bulge (BHB) motif, that defines the intron-exon boundaries. The eukaryal enzyme normally acts in a mature-domain dependent mode (MDD); the enzyme recognizes a tripartite set of RNA elements. One subset of recognition elements is localized in the mature domain, while two other subsets are localized at the exon-intron boundaries. A pivotal role is played by a base-pair located near the site of 3' cleavage, the so-called anticodon-intron pair (A-I pair). A purine is strongly preferred at the position preceding the 5' cleavage site.

The primary and secondary structures at the exon-intron junctions of the archaeal and eukaryal pre-tRNAs do not show evident similarities, with the exception of the three-nucleotide bulged structure, closed by the A-I pair and containing the 3' cleavage site, that resembles half of the BHB. The endonuclease are evolutionarily related ( ), but their substrate recognition properties appear drastically different. It has previously been shown, however, that the *Xenopus* and the yeast endonucleases retain the ability to operate in the MDI mode.

### BRIEF SUMMARY OF THE INVENTION

In one embodiment, the present invention is an *in vitro* method of cleaving a target RNA molecule comprising the steps of exposing the target molecule in a cell to heterologous archaeal tRNA splicing endonuclease, wherein the target molecule is in a bulge-helix-bulge conformation created by two mRNA molecules, wherein the two mRNA molecules are the target RNA molecule and a second RNA molecule, and cleavage occurs between second and third nucleotides at bulges in the bulge-helix-bulge conformation and cleavage products are generated, wherein the target molecule does not comprise a tRNA structure, and ligating the cleavage products from the target RNA and the second RNA so that a fusion RNA is formed comprising at least one cleavage product from the first target RNA molecule and at least one cleavage product from the second target RNA molecule.

Preferably the method of the invention is carried out in a cell which is a mammalian, plant or eubacteria cell. Preferably this is a eukaryotic, preferably mammalian, cell.

Other features, objects and advantages of the present invention will be apparent to one of skill in the art after review of the specification and claims.

### BRIEF DESCRIPTION OF THE SEVERAL VIEWS OF THE DRAWING

Fig. 1A depicts the A-I interaction. Fig. 1B depicts a substrate cleaved in both the mature-domain dependent and the mature-domain independent modes.

Fig. 2A shows the *Xenopus* endonuclease can cleave *in vivo* in the mature-domain independent mode. Fig. 2B shows that the yeast endonuclease mutant sen2-3 cleaves pre-tRNA^{Archeuka} at both sites. Fig. 2C is a comparison of models of enzyme-substrate interaction.

Fig. 3 depicts cleavage of a non-tRNA molecule by the *Xenopus* endonuclease.

Fig. 4 is a diagram of BHB-mediated trans-splicing.

Fig. 5 describes a scheme of BHB (bulge-helix-bulge) insertions in the GFP mRNAs. The upper and lower parts of the figure show the structure of the GFPof and the GFP-BHB genes, as well as the BHB position. In the middle, the different BHB substrates are detailed. The BHB and the BHB-stop are processed by the MJ-endoribonuclease whereas the BHB+3 is MJ-endoribonuclease insensitive. Intronic ribonucleotides are underlined, stop codons blocking all reading frames are in bold face and the base-pair insertion disrupting the canonical BHB structure is boxed in green.

Fig. 6 depicts direct GFP and Hoechst fluorescence of NIH3T3 transiently transfected with different plasmids as indicated in the figure. Transient transfections were performed with (a) 0.5 µg pEGFP-N3 plus 1.5 µg pMJ; (b) 0.5 µg pGFP of plus 1.5 µg of pMJ; (c) 0.5 µg pGFPof plus 1.5 µg put-MJ; (d) 0.5 µg pGFP of +3 plus 1.5 µg pMJ.

Fig. 7 depicts splicing analysis of mRNAs deriving from NIH3T3 cells transfected with pMJ or put-MJ plasmids, and pGFP of, pGFPof+3 and pGFPof-STOP target constructs. Fig. 7A mRNA structure of pGFPof, PGFPof+3 and pGFPof-STOP target constructs and the PCR primers position. Fig. 7B RT-PCR analysis of different GFP mRNA in the presence or absence of functional MJ enzyme. NIH3T3 were transiently transfected with different plasmids as indicated in the figure. A constant 1:3 molar ration between plasmids coding for target tRNAs and for MJ-endoribonuclease was used.

Fig. 8 is a sequence analysis of RT-PCR products derived from NIH3T3 cells transiently transfected with pGFPof and pMJ plasmids. Sequences were performed using RT-PCR products eluted from a gel. In the middle, the arrows indicate the intron-flanking nucleotides.

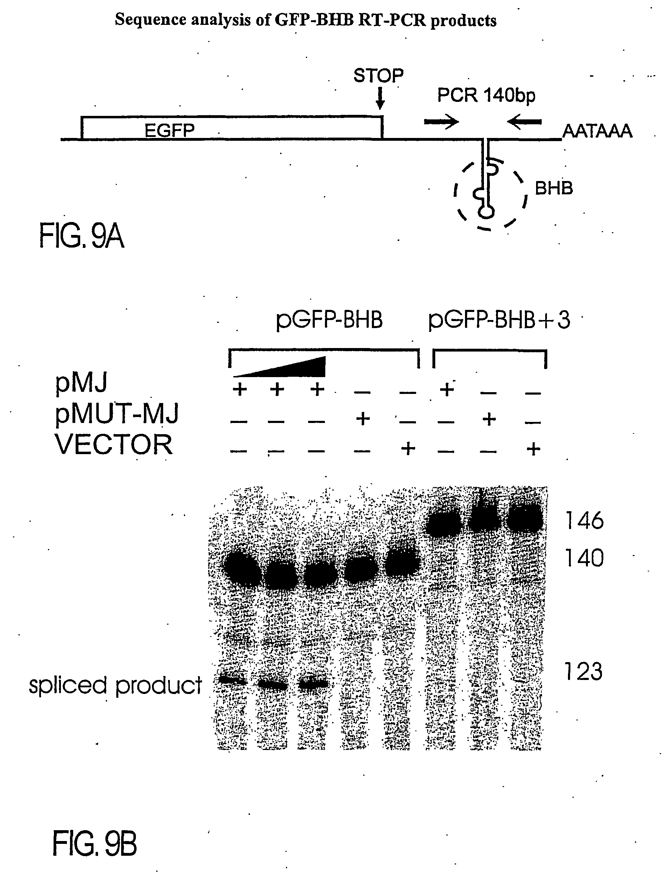

Fig. 9 is a splicing analysis on mRNAs derived from NIH3T3 cells transfected with pMJ or put-MJ plasmids, and pGFP-BHB or pGFP-BHB target constructs. Fig. 9A: mRNA structure of pGFP-BHB+3 target constructs and the PCR primers position. Fig. 9B: RT-PCR analysis of different GFP mRNA in the presence or absence of functional MJ enzyme. NIH3T3 were transiently transfected with different plasmids as indicated in the figure. A constant 1:3 molar ratio between plasmids coding for target TRNAS and for MJ-endoribonuclease was used in lanes 3-8. In lanes 1 and 2 ratios of, respectively, 3:1 and 1:1 were used instead.

Fig. 10 is a sequence analysis of RT-PCR products derived from NIH3T3 cells transiently transfected with pGF-BHB and pMJ plasmids. Sequences were performed using RT-PCR products eluted from a gel. In the middle, the arrows indicate the intro-flanking nucleotides.

### DETAILED DESCRIPTION OF THE INVENTION

The present invention uses a method of RNA cleavage involving substrate recognition by tRNA splicing endonucleases. Example 1 describes features required for eukaryotic endonuclease cleavage. Example 2 describes *in vitro* experiments designed to cleave a mouse mRNA. Example 3 describes cleavage performed in cells in culture and re-ligation of mRNAs in mouse cells.

### RNA Cleavage by tRNA Splicing Endonucleases

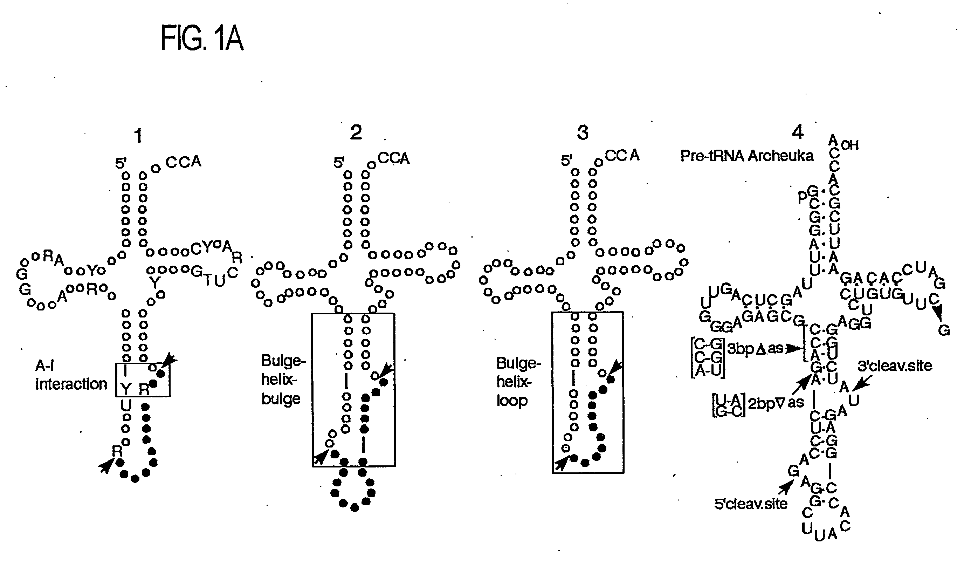

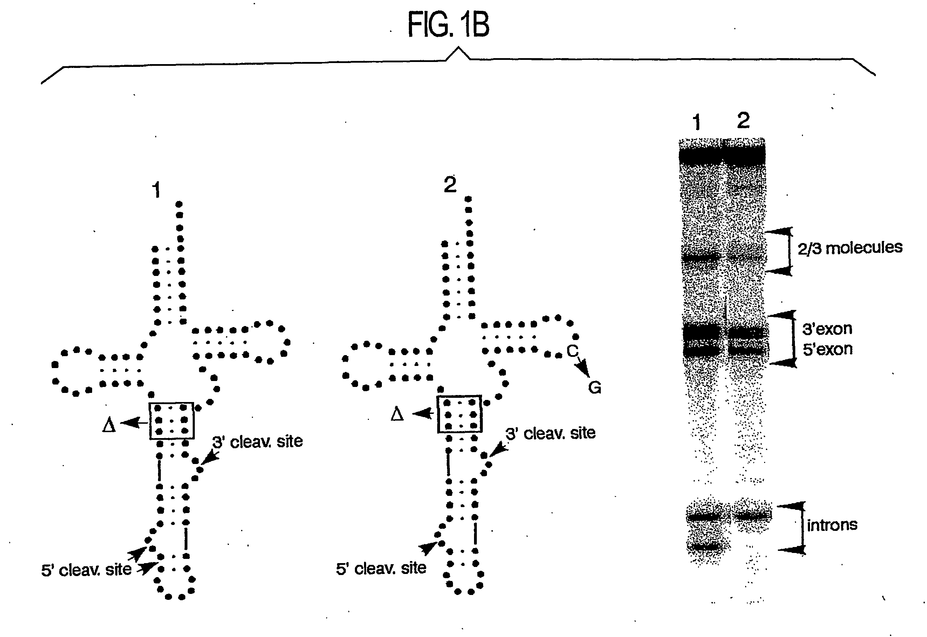

In the present invention a double-stranded RNA molecule is cleaved, wherein the molecule has assumed a bulge-helix-bulge (BHB) conformation. (For a more complete understanding of the structural requirements for the BHB conformation, one should closely examine all figures.) The bulge-helix-bulge conformation comprises an RNA bulge on one strand, a 4 base-pair helix, and an RNA bulge on the opposite strand. The bulges are typically 3 nucleotides in length and the cleavage sites are as described in Fig. 1. In general, the BHB is cleaved (going in the direction 5' - 3') at the bond between the second and third nucleotide at the bulges.

In one method of the present invention, a BHB-containing RNA is exposed to a tRNA splicing endonuclease from archeobacteria. The cleavage reaction can be performed in a cell *in vitro* (see Materials and Methods). While the material presented below in the Examples describes certain preferable and typical cleavage reactions, one of skill in the art will know that modifications in reaction conditions, such as enzyme and substrate concentration and buffer and reaction condition components, would be modified to produce successful cleavage reactions.

The cleavage is in the absence of the mature domain of the tRNA structure or sequence. The molecule to be cleaved does not require the D, T and acceptor arm of tRNA structure.

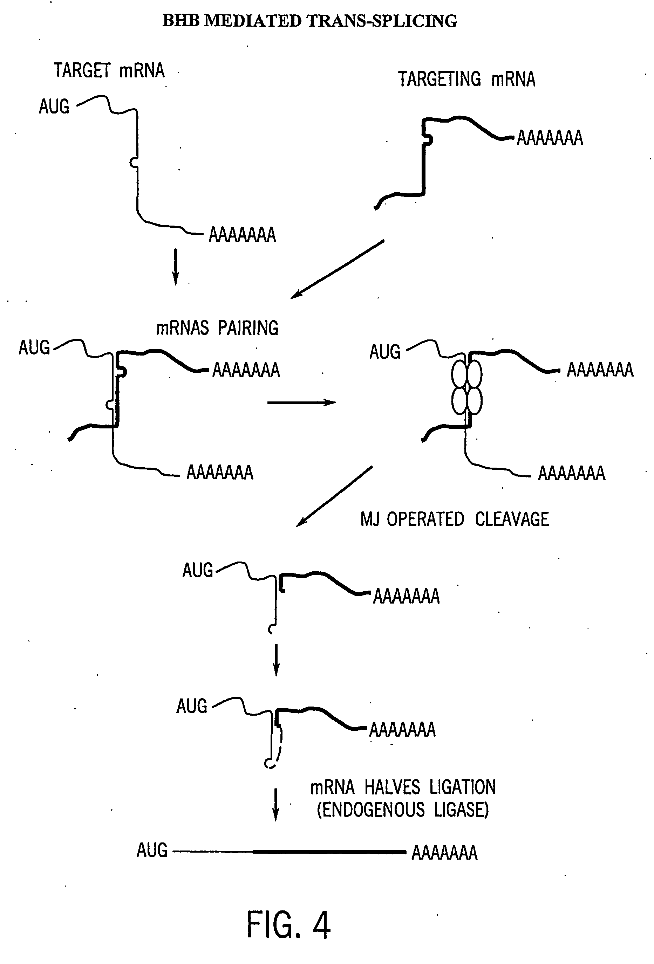

The BHB could result by the folding of an RNA molecule (BHB in cis) or it could be generated using two different RNA molecules (BHB in trans) (see Fig. 4). One could also design an oligonucleotide capable of forming a BHB structure when hybridized to a target RNA. Therefore, in principle one may target the cleavage site to any desired location on an RNA molecule.

### Creation of Oligonucleotides

One of skill in the art of molecular biology would understand how to create an oligonucleotide that would result the bulge-helix-bulge conformation and appropriate splicing site. Preferably, such an oligonucleotide would be at least between 50 and 70 nucleotides long. Most preferably, the oligonucleotide would be between 58 and 62 nucleotides. In this oligonucleotide, at least approximately 25 (± 5 nucleotides) nucleotides would be needed on either side of the bulge-helix-bulge conformation.

The oligonucleotide is preferably RNA or a modified RNA molecule. However, we envision that a DNA oligonucleotide would also be suitable.

### Selection of tRNA Splicing Endonuclease

In the present invention, one would use in *in vitro* experiments archaeal tRNA splicing endonucleases (preferably *M. jannaschii*). In cells (preferably mammalian cells) one would preferably utilize endogenous tRNA splicing endonucleases and express one of a battery of archaeal enzymes (*Archeoglobus fulgidus, Pyrobaculum aerophilum, Halobacterium* sp. NRC-1, *Methanocuccus jannaschii*). Typically the archaeal enzymes can be expressed in a mouse in a conditional fashion (space and time) utilizing specific mouse promoters.

### Preferred Applications

One could construct a BHB in *trans* with any cellular RNA and, therefore, provide cleavage at a desired sequence. Preferred applications include using the RNA cleavage method to cut a target RNA into defined ends with a 2',3' cyclic phosphate capable of being ligated. One would also use the present invention to degrade particular targeted RNAs.

One could use the present invention to demonstrate the presence of particular RNAs. For example, one could label an oligonucleotide, wherein the oligonucleotide is capable of forming the BHB structure with the RNA target, and look for cleavage products after the duplex has been exposed to the endonuclease. This may be by means of FRET (fluorescence resonance energy transfer), where one would observe a fluorescent signal if the two ends of a fluorescently labeled oligonucleotide probe were separated.

Therapeutically, one could use the method of the present invention to treat or remove unwanted RNAs from cells. Particular examples would be viral RNAs. In one embodiment, one would express oligonucleotide probes, wherein the probe is capable of forming the BHB structure with a target RNA, into the desired cell. Endogenous tRNA splicing endonucleases or archaeal enzymes expressed in mammalian cells would then cleave the target RNA.

In a preferred form of the present invention, one would avoid the presence of ADAR enzymes ("Adenocine Deaminases Acting on RNA") by either providing a substitute ADAR substrate (such as excess double-stranded RNA) or designing a target oligonucleotide that is appropriate for cleavage by the tRNA splicing endonuclease but not cleavage by the ADAR.

### Use of Archaeal Enzymes in in vivo Cleavage and Formation of Fusion RNAs

In the present invention, two different RNAs are cleaved as described above, and the fragments re-ligated. This embodiment is described in Fig. 4 of the present invention. Typically, one would cotransfect a cell with two plasmids: one coding for an RNA that could form a BHB in trans with another RNA coded by the second plasmid. One could *in vivo* conditionally (space and time) activate an mRNA containing a BHB inserted in the coding sequence. Excision of the intron and ligation reconstitutes the correct reading frame.

In a preferred form of this embodiment, the cleavage and formation of fusion RNA is within a cell selected from mammalian cells, plant cells and eubacterial cells. Preferably, the cleavage and re-ligation is within mammalian cells.

The Examples below describe a preferred manner of setting up such an *in vivo* embodiment. Of course, one of skill in the art would understand that modifications in enzyme and substrate concentration and reaction conditions would be within the scope of the invention and not affect the ultimate creation of cleavage products and formation of fusion RNA.

### EXAMPLES

### Materials and Methods: Targeted RNA Cleavage by tRNA Splicing Endonuclease (Examples 1 and 2)

### Amplification

PCR was performed in 100 µl reaction containing 5-10 fmole of template, 100 µM of each primer, 125 µM dNTP, 1.5 mM MgCl₂, 50 mN KCI, 10 mM Tris-HCl pH 8.2, 5 U of native Taq Polymerase (Perkin-Elmer) under the following conditions: 95°C 30 seconds, 55°C 10 seconds, 72°C 2 minutes for 18 cycles. The DNA was phenol extracted and ethanol precipitated.

### Transcription

Transcription of templates by T7 RNA polymerase was performed in 40 mM Tris HCl pH 8.0, 6 mM MgCl₂, 10 mM dithiothreitol, 2 mM spermidine, 500 µM ATP, CTP and GTP and 100 µM UTP, 2.5 mM 5' GMP, 2.5 µM [α³²P] UTP (800 Ci/mmole, Amersham) using 5 U/µl T7 RNA polymerase (Pharmacia), 0< 1 U/µl RNAguard (Pharmacia) at 37°C for 90 minutes. After phenol extraction and ethanol precipitation, transcripts were purified on a 8% denaturing acrylamide gel, eluted and ethanol precipitated.

### Endonuclease assay

Labeled precursors (0.7 nM) were incubated in 30 µl reaction mixture containing 10 mM HEPES pH 7.5, 7 mM MgCl₂, 70 mM NH₄Cl, 2.5 mM dithiothreitol, 10% glycerol (v/v) with 10 µl of purified endonuclease at 22°C for 30 minutes. Cleavage products were analyzed by gel electrophoresis on 12% denaturing polyacrylamide gels.

### tRNA synthesis

Pre-tRNA^{Phe} and pre-tRNA^{Phe(U-AG-C)_{∇}}were synthesized as described (E. Mattoccia, et al., Cell 55:731-738, 1988; M.I. Baldi, et al., Science 255:1404-1408, 1992; E. Di Nicola Negri, et al., Cell 89:859, 1997). Templates for the synthesis of the Archaeuka precursors were constructed by polymerase chain reaction (PCR). The PCR templates were the full-length pre-tRNAs. One primer contained the T7 promoter and part of the 5' exon. The other was comprised of the desired sequence of the 3' exon. Conditions for PCR, transcription by T7 RNA polymerase, and endonuclease assays were as described (E. Mattoccia, et al., supra, 1988; M.I. Baldi, et al., supra,1992; E. Di Nicola Negri, et al., supra, 1997). *Xenopus laevis* endonuclease was purified as in D. Gandini Atardi (D. Gandcini Atardi, et al., Methods Enzymol. 181:510-517, 1989).

### Transcription

We utilized Ambion Megascript kits.

Transcription products were purified on 8 M urea polyacrylamide gels. After elution the products were alcohol precipitated.

### Annealing to produce dsRNA

Annealing was at 60' at 68°C in 50 mM TRIS-HCl pH 7 300 mM NaCl 2 mM EDTA, with slow cooling to 35°C, purification on polyacrylamide gels, elution and alcohol precipitation. The concentration of each strand was 0.01 mg/ml.

### X.I. Endonuclease assay

The assay was for 90' at 25°C in 30 ml total volume 10 mM Hepes pH 7.5, 70 mM NH₄Cl 7 mM MgCl₂m 2,5 nN DTT, 10% glycerol, substrate 2 nM.

### M.J. Endonuclease assay

The assay was for 30' at 65°C in 30 ml total volume 40 mTRIS-HCl pH 7.5 5mM MgCl₂, 10% glycerol, 50 mM, substrate 2 nM.

Incubation mixtures were treated with SDS, prot K and phenol, followed by alcohol precipitation. Products were analyzed in 8 M urea polyacrylamide gels. Samples were treated with urea for 5' at 65°C or with formamide for 5' at 95°C.

### Example 1

Eukaryal tRNA splicing endonucleases use the mature domains of pre-tRNAs as their primary recognition elements. However, they can also cleave in a mode that is independent of the mature domain, when substrates are able to form the bulge-helix-bulge structure (BHB), which is cleaved by archaeal tRNA endonucleases. We present evidence that the eukaryal enzymes cleave their substrates after forming a structure that resembles the BHB. Consequently, these enzymes can cleave substrates that lack the mature domain altogether. That raises the possibility that these enzymes could also cleave non-tRNA substrates that have a BHB. As predicted, they can do so both *in vitro* and *in vivo.*

### Introduction

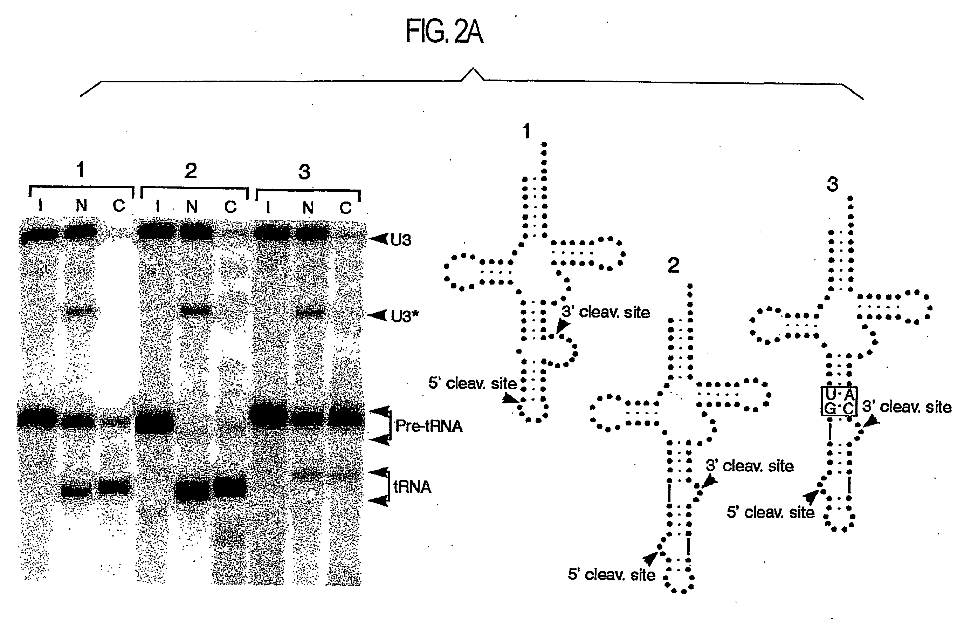

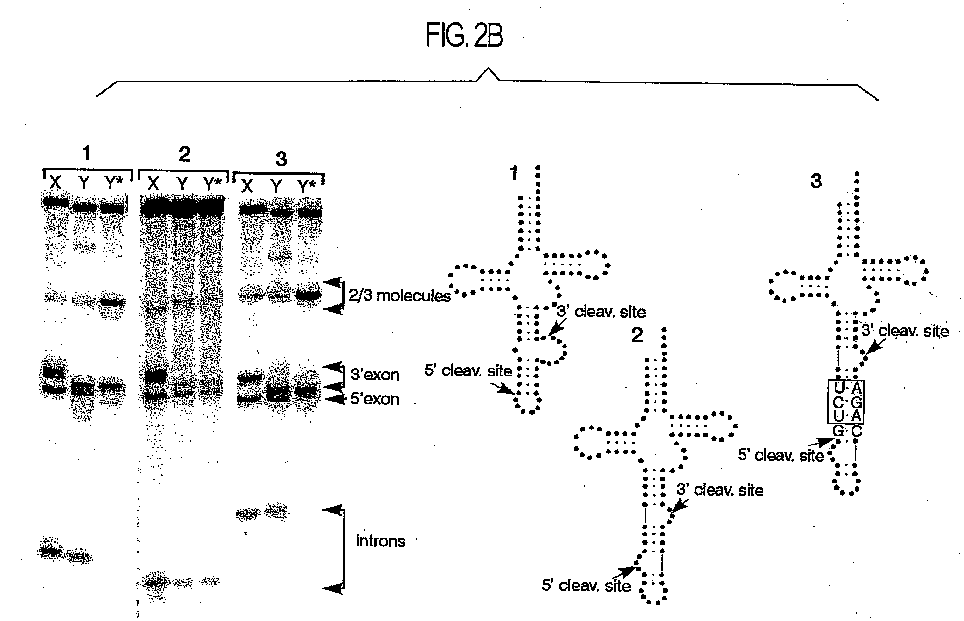

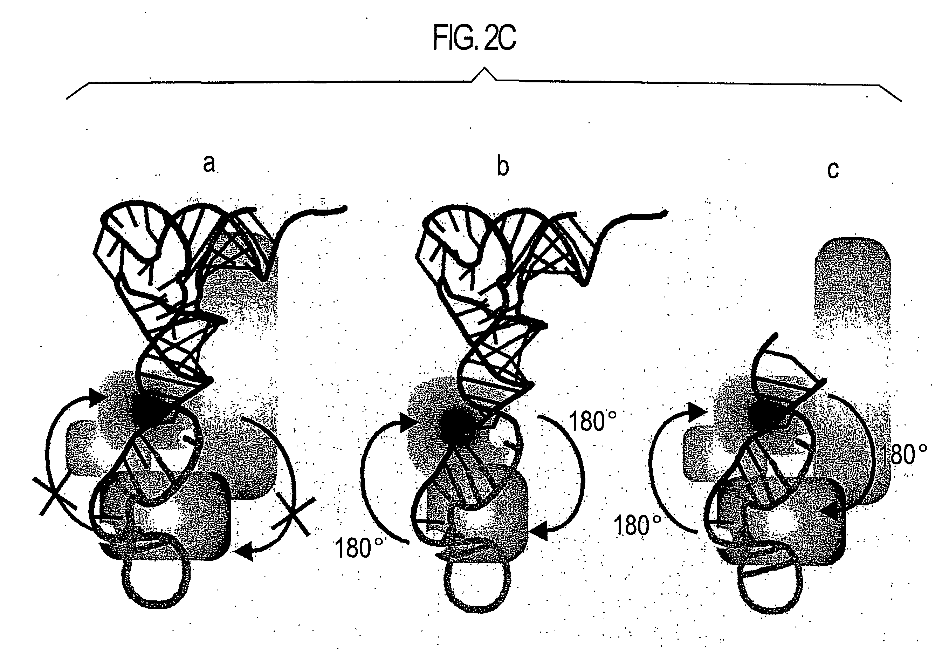

Accuracy in tRNA splicing is essential for the formation of functional tRNAs, and hence for cell viability. In both Archaea and Eukarya the specificity of splicing resides in recognition of tRNA precursors by tRNA splicing endonucleases (Belfort and Weiner, Cell 89:1003-1006, 1997; Trotta and Abelson, The RNA World, pp. 561-583, 1999). Archaeal tRNA splicing endonucleases cleave pre-tRNAs only using an RNA structure comprised of two bulges of three nucleotides each (where cleavage occurs) separated by four base pairs. This structure, called the bulge-helix-bulge (BHB) (Fig. 1A, 2) (Daniels, et al., J. Biol. Chem. 260:3132-3134, 1985; Diener and Moore, Mol. Cell. 1:883-894, 1998), functions independently of the part of the molecules that constitutes the mature tRNA, so we refer to this type of recognition of the cleavage sites as being the mature-domain independent mode.

In contrast, eukaryal tRNA splicing endonucleases require interaction with the mature tRNA domain for orientation, so we refer to that recognition as the mature-domain dependent mode (Mattoccia, et al., Cell 55:731-738, 1988; Reyes and Abelson, Cell 55:719-730, 1988).

Recognition of pre-tRNAs by eukaryal tRNA splicing endonucleases normally requires the mature tRNA domain, as well as a base-pair, called the anticodon-intron (A-I) pair (Fig. 1A), which is formed between nucleotides in the anticodon loop and the intron (Baldi, et al., Science 255:1404-1408, 1992). The A-I pair must be at a fixed distance from the mature domain for cleavage to occur and cleavage near this base pair generates the 3' splice site. An independent cleavage event, also at a fixed distance from the mature domain (usually at a purine), generates the 5' splice site.

The two modes of substrate recognition are characterized by two distances. In the mature-domain independent mode the helix of the BHB sets the distance between the two bulges; in the mature-domain dependent mode the distance is fixed relative to reference in the mature domain.

While the subunit structures of the eukaryal and archaeal enzymes differ significantly (Trotta and Abelson, supra, 1999), as do the superficial structures of the cleavage sites, we have demonstrated that both the *Xenopus* and yeast tRNA splicing endonucleases can operate in the mature-domain independent mode, characteristic of Archaea (Fabbri, et al., Science 280:284-286, 1998). The results reported in ths paper explain why the eukaryal endonucleases retain the ability to operate in the mature-domain independent mode when their natural substrates do not have a BHB.

### Results and Discussion

Fig. 1A depicts the A-I interaction. A conserved purine residue in the intron three nucleotides from the 3' cleavage site (molecule 1, R in box) must pair with a pyrimidine in the anticodon loop 6 nucleotides upstream of the 5' cleavage site (molecule 1, Y in box) to form the A-I (for anticodon-intron) interaction (Baldi, et al., supra, 1992). BHB (molecule 2): Two bulges of three nucleotides each (where cleavage occurs) rigidly separated by four base pairs (Daniels, et al., supra, 1985; Diener and Moore, supra, 1998). BHL (molecule 3): A three-nucleotide 3' site bulge, a four base-pair helix and a loop containing the 5' site. Pre-tRNA^{Archeuka} and its variants (molecule 4): The hybrid pre-tRNA molecule pre-tRNA^{Archeuka} is a substrate for both the eukaryal and archaeal endonucleases. It consists of two regions derived from yeast pre-tRNA^{Phe} (nucleotides (nt) 1-31 and not 38-76) joined by a 25 nt insert that corresponds to the BHB motif of the archaeal pre-tRNA^{Trp}. It has a typical eukaryal mature domain with cleavage sites located at the prescribed distance from the reference elements and a correctly-positioned A-I base pair, all of which should ensure correct recognition by the eukaryal endonuclease when the enzyme operates in the mature-domain dependent mode. In addition, the presence of the BHB motif confers substrate characteristics that are recognizable by the eukaryal enzyme when it operates n the mature-domain independent mode. Fig. 1B depicts a substrate cleaved in both the mature-domain dependent and the mature domain independent modes. Products of digestion by the *Xenopus* tRNA splicing endonuclease. Molecule 1: Pre-tRNA^{Archeuka} 3bpΔas; molecule 2: Pre-tRNA^{Archeuka} 3bpΔas, C56G.

Fig. 2A shows that the *Xenopus* endonuclease can cleave *in vivo* in the mature-domain independent mode. Low amounts of ³²P-labeled RNAs corresponding to Pre-tRNA^{Pne} (1); Pre-tRNA^{Archeuka} (2) and Pre-tRNA^{Archeuka} 2bp∇as (3) were injected into nuclei of *Xenopus* oocytes and 2 hours later the intracellular distribution of the injected primary pre-tRNA transcripts and of the mature tRNAs were determined by analysis of total nuclear (N) and cytoplasmic (C) RNAs. The injected precursor RNAs in the cytoplasm probably resulted from inefficient nuclear retention, I., input.

Fig. 2B shows that the yeast endonuclease mutant sen2-3 cleaves pre-tRNA^{Archeuka} at both sites. Molecule 1, pre-tRNA^{Phe}; molecule 2, pre-tRNA^{Archeuka}; molecule 3, Pre-tRNA^{Archeuka} 3bp∇BHB, C8G, G24C, X. *Xenopus laevis;* Y, yeast *S. cerevisiae* wild-type; Y* yeast *S. cerevisiae* sen2-3. The sen2-3 preparation was contaminated with a 3'exonucleolytic activity that partially degraded the 3' end of the precursor, reducing the size of the 3' half product. The sequences of the products of the intron excision reaction have been verified by fingerprinting (data not shown). In the sen2-3 mutant, one residue in loop L7, Gly²⁹², is changed to Glu (Trotta and Abelson, supra, 1999). Loop 7 contains a histidine residue that is absolutely conserved in all tRNA endonucleases, and that probably acts as a general base by deprotonating the nucleophile 2'-hydroxyl group (Trotta and Abelson, supra, 1999). The residues on loop 7 immediately surrounding the conserved histidine residue are not conserved among the tRNA endonucleases. We suggest that these residues have a role in the restructuring of the 5' cleavage site in the eukaryal enzymes.

Fig. 2C is a comparison of models of enzyme-substrate interaction: (a) Pre-tRNA^{Archeuka} Eukaryal enzyme (Trotta and Abelson, supra, 1999), (b) Pre-tRNA^{Archeuka} Archaeal enzyme (Trotta and Abelson, supra, 1999), and (c) BHB Eukaryal enzyme.

A proposal for loss of symmetry during evolution of the intron excision reaction. In Archaea, the recognition element in pre-tRNA is the BHB, which has pseudo-two-fold symmetry (Diener and Moore, supra, 1998; Trotta and Abelson, supra, 1999). Since the endonuclease does not bind to the mature domain of pre-tRNA, the enzyme is oriented in such a ways that both active sites can cleave either of the intron-exon junctions (b). The primary recognition element of the eukaryal endonuclease, on the other hand, is the asymmetrically located mature domain of pre-tRNA; interaction with that domain imposes an orientation of the enzyme on the substrate, so that each active site is specific to one or the other intron-exon junctions. In the absence of a mature domain, as with the mini-BHB (Febbri, et al., supra, 1998), the eukaryal enzyme is free to recognize pseudo-two-fold symmetric elements in the substrate, so that both active sites in the enzyme can bind to either junction (c). However, when a substrate has both a mature domain and a symmetric BHB, as in Pre-tRNA^{Archeuka}, the eukaryal endonuclease can interact with the mature domain, and the added energy of this binding would be likely to orient the enzyme (a).

We propose that during evolution, once endonucleases were able to recognize the mature domain, the need for a symmetric BHB recognition site diminished; however, the active sites of the eukaryal enzymes were maintained, allowing them to cleave pre-formed BHB structures. Because the orientation of the two cleavage sites in the enzyme remained constant, the eukaryal pre-tRNAs had to maintain the ability to form a BHB-like structure upon binding the eukaryal enzyme; part of that requirement is seen in the -A-I base pairing rule and in the BHL.

The artificial substrate Pre-tRNA^{Archeuka} contains both a mature domain and a BHB (Figs. 1A, 2). The eukaryal enzymes cleave the substrate with a two base-pair insert in the anticodon stem, 2bpVas (Figs. 1A, 4), only in the mature-domain independent mode (Fabbri, et al., supra, 1998). The sites of cleavage by the eukaryal enzyme are fixed by recognition of local BHB structure rather than by reference to the mature domain.

The *Xenopus laevis* endonuclease can also cleave *in vivo* in the mature-domain independent mode. When Pre-tRNA^{Archeuka} and pre-tRNA^{Archeuka} 2bpVas were injected into *Xenopus* oocyte nuclei, both substrates were spliced and ligated. The size of the mature pre-tRNA^{Archeuka} 2bp∇as, which is four bases longer than mature wild-type pre-tRNA^{Phe}, indicates that the *Xenopus* enzyme cleaves in the mature-domain independent mode *in vivo* just as it does *in vitro* (Fig. 2A). The substantial amounts of each precursor exported before cleavage probably results from saturation of nuclear retention (Arts, et al., EMBO J. 17:7430-7441, 1998; Lund and Dahlberg, Science 282:2082-2085, 1998).

Some substrates are cleaved in both modes. Pre-tRNA^{Archeuka} 3bpΔas (Fig. 1B), which has a three base-pair deletion in the anticodon stem, is cleaved in both modes. In this case, the two modes yield distinct product sizes, and both are observed. Two introns are visible in Fig. 1B, lane 1. One of the products is not produced in the C56G mutant reflecting the inability of the enzyme to cleave a substrate that cannot form a normal mature domain in the mature-domain dependent mode.

We now propose that the orientation of the substrate in the active site of the eukaryal enzyme requires the formation of a structure that resembles a BHB; the A-I pair would play a pivotal role in this process, as it represents the closing base pair of one of the bulges. This model predicts that recognition of the mature tRNA domain by a eukaryal tRNA splicing endonuclease allows subsequent formation of a BHB-like cleavage structure.

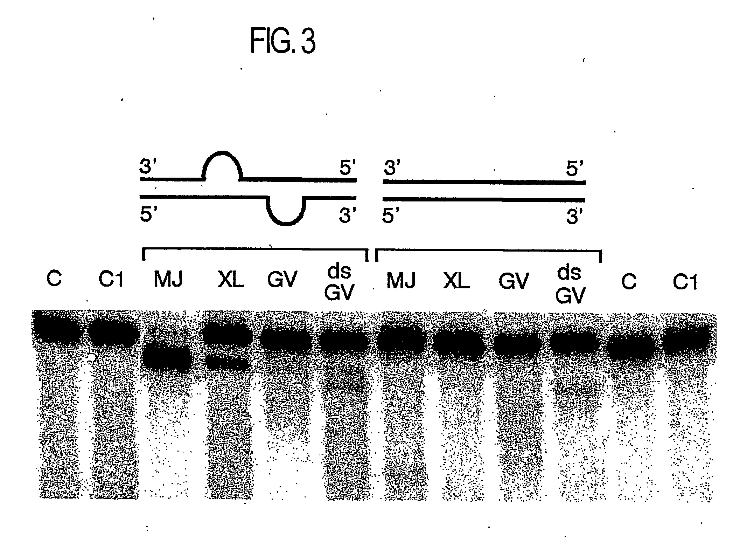

In addition to the A-I pair (Baldi, et al., supra, 1992), other relics of the archaeal world provide insight into the mechanism of the eukaryal cleavage reaction. Some eukaryal pre-tRNAs present motifs that resemble the BHB. The sequence of the *Caenorhabditis elegans* genome shows the tRNA genes corresponding to three isoacceptor species (Leu, Tyr, Ile) contain introns (The C. elegans Sequencing Consortium, 1998). The nematode pre-tRNAs present a motif which we call BHL (Figs. 1A, 3), which resembles the BHB in that it has the 3' site bulge and the four base-pair helix but the 5' site is in a loop rather than in bulge. These three intron-containing pre-tRNAs of *C. elegans* are cleaved correctly by both yeast and *Xenopus* endonucleases, as well as the *Parascaris equorum* tRNA splicing endonuclease, but not by the archaeal enzyme (data not shown). Thus, the only truly universal substrate is an RNA with a BHB (Fabbri, et al., supra, 1998).

Because they can both cleave the BHB, the archaeal and eukaryal endonucleases are likely to have identical dispositions of active sites, a feature conserved since their divergence from a common ancestor (Trotta and Abelson, supra, 1999; Fabbri, et al., supra, 1998) (Fig. 2C). We suggest that the mature-domain dependent mode arose through specialization of the subunits of the eukaryal enzyme.

We propose that the eukaryal enzymes possess a mature-domain dependent 5'site restructuring activity (Di Nicola, et al., Cell 89:859-866, 1997). Such an activity would be required for the last steps of substrate recognition by the eukaryal enzymes, recapitulating the recognition process of their archaeal counterparts. The 5' site restructuring activity is not needed to cleave the BHB because it already has a correctly structured 5' site; however the activity is responsible for improving the efficiency of cleavage at the 5' site in BHL (P. Fruscoloni, M. Zamboni, M.I. Baldi and G.P. Tocchini-Valentini, manuscript in preparation). The Ascaris enzyme also has a mature-domain dependent 5' restructuring activity, but it differs from that of *Xenopus* because it is unable to restructure a typical eukaryal pre-tRNA such as yeast pre-tRNA^{Phe} (P. Fruscoloni, M. Zamboni, M.I. Baldi and G.P. Tocchini-Valentini manuscript in preparation).

Our model predicts the existence of mutants of the eukaryal enzyme that lack the 5' restructuring activity. Such mutants would be unable to cleave a eukaryal pre-tRNA at the 5' site, but could cleave at the 3' site. More importantly, these restructuring mutants should cleave precursors that already have a BHB.

The yeast endonuclease is an αβγó heterotetramer (Trotta, et al., Cell 89:849-858, 1997). Homology relationships and other evidence suggest that two subunits of the enzyme, Sen2p and Sen34p, contain distinct active sites, one for the 5' site, the other for the 3' site. The mutant sen2-3 is defective in cleavage of the 5' site (Ho, et al., EMBO J. 9:1245-1252, 1990); Fig. 2B shows that sen2-3 extracts cleaves the 3' but not the 5' site of yeast pre-tRNA^{Phe}. The same extract, however, cleave Pre-tRNA^{Archeuka} at both sites (Fig. 2B). Thus, sen2-3 cleaves the 3' but not the 5' sites in substrates lacking a BHB, as would be expected if it lacked the mature-domain dependent 5' site restructuring activity. This conclusion is reinforced by the fact that pre-tRNA^{Archeuka} 3bpVBHB, a substrate that can interact with the enzyme only in the mature-domain dependent mode, is cleaved only at the 3' site (Fig. 2B).

It is unlikely that the lack of cleavage at the 5' site of pre-tRNA^{Phe} results simply from inactivation of the catalytic site since the mutated amino acid is not near this site, based on the crystal structure of the enzyme (Li, et al., Science 280:279-284, 1998). Moreover, Pre-tRNA^{Archeuka}, which has a BHB, is cleaved at both sites even though its mature domain should prevent binding of the sen34 active site to the 5' site (Fig. 2B). Unfortunately, expression of a sen2-3 and sen34 double mutant enzyme is very likely to be lethal, making production of a doubly mutated enzyme impossible.

### Example 2

The ability of the eukaryal enzyme to recognize and cleave independently of the mature domain creates the possibility for cleavage of non-tRNA substrates (Fig. 3). If the eukaryal endonuclease can recognize and cleave substrates in the mature-domain independent mode, any RNA that contains a BHB structure should be able to serve as a substrate. Such a target could be generated in mRNA by adding a suitable RNA oligonucleotide.

Fig. 3 depicts cleavage of a non-tRNA molecule by the *Xenopus* endonuclease. Profilin I mRNA duplexes (cartoon) consisting of ³²P-labeled sense strand and cold antisense strand (0.6 nM) were incubated with *Methanococcus jannaschii* endonuclease (MJ for 30 minutes at 65°C); *Xenopus laevis* endonuclease (XL for 90 minutes at 25°C); germinal vesicles extract (GV for 90 minutes at 25°C). The reacted RNA was treated as described (Mattoccia, et al., supra, 1988; Baldi, et al., supra, 1992; Fabbri, et al., supra, 1998) and analyzed in 8M urea polyacrylamide gels. Two fragments were generated from profilin I mRNA (417 nts and 53 nts). The gel shows only the larger fragment. Unrelated ³²P-labeled dsRNA (low specific activity) was added where indicated (the concentration was 300x that of the profilin I duplex). C, duplex containing the BHB; C1, full duplex.

Fig. 3 shows that the archaeal and eukaryal enzymes cleave mouse profilin 1 mRNA (Widada, et al., Nucleic Acids Res. 17:2855, 1989), when the RNA is complexed with another oligoribonucleotide forming a BHB. This cleavage occurs in a BHB-dependent manner because fully double-stranded molecules (Fig. 3) and molecules presenting an insertion of three base pairs in the helix of the BHB are not cleaved (data not shown). Fig. 3 shows that cleavage also occurs in extracts of germinal vesicles (GV extracts). Again, cleavage is BHB-dependent. However, cleavage in this extract occurred only in the presence of a 100-fold excess of unrelated double-stranded RNA (dsRNA). Pre-tRNA^{Archeuka}, on the contrary, is cleaved at high efficiency (data not shown). An explanation for these differences is the presence in GV extracts of adenosine deaminases (ADARs) that convert adenosines to inosines within dsRNA (Bass and Weintraub, Cell 35:1089-1098, 1988), thereby causing the RNA duplex to fall apart, disrupting the BHB structure. Presumably, at low concentration of dsRNA, ADARs deaminate the substrate and, as a result of the increased single-stranded character of the molecule, the BHB is destroyed.

Our results indicate that the formation of a BHB is an obligate step in cleavage by the eukaryal endonucleases and explain why the eukaryal endonucleases retain the ability to operate in the mature-domain independent mode when their natural substrates do not have a BHB.

### Example 3

### RNA Engineering

Various approaches are currently being used to elucidate the principles that underlie the construction and function of eukaryotic cells and organisms. Some of these approaches, such as gene targeting or the gene trap technology, operate at the level of the DNA. Others, like the Tet system, regulate gene expression at the level of transcription. Yet another kind of approach, such as induced dimerization, works at the protein level.

It is becoming clear that processes occurring at the RNA level such as, for example, alternative splicing and editing, play an extremely important role in expanding protein diversity and might therefore be partially responsible for the apparent discrepancy between gene number and complexity. Developing a full catalogue of transcripts corresponding to a single gene and determining each of their functions will be a major challenge of the proteomic era. What is needed is a technology that makes RNA engineering possible.

The BHB is recognized and cleaved by archaeal and eukaryal tRNA endonucleases. It is therefore possible to generate non-tRNA substrates for the enzymes; any RNA that contains a BHB structure is capable of serving as a substrate. Such a target can be generated in mRNA either by inserting a BHB in cis in the coding or untranslated regions, or by adding a suitable RNA oligonucleotide to form a BHB in trans.

In mouse cells, we have found that the halves generated by cleavage of BHB in cis in a mRNA molecule are ligated by an endogenous ligase activity. Fig. 4 shows that, when the BHB is formed in trans by two different RNA molecules, a fusion mRNA may result.

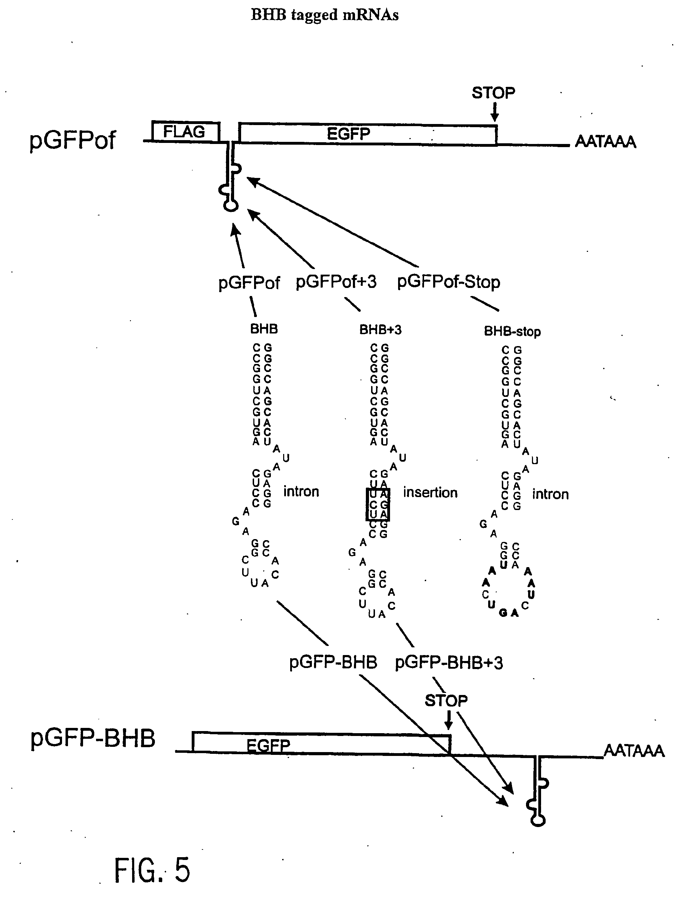

A sequence was inserted in the coding region of the GFP gene so that the transcription of the coding strand yields an mRNA harbouring a canonical BHB (Fig. 5). The mutated GFP gene was named "GFPof," since the presence of the intron in the messenger renders the latter out of frame. Production of GFP requires that the intron be precisely excised and that the exons be subsequently ligated.

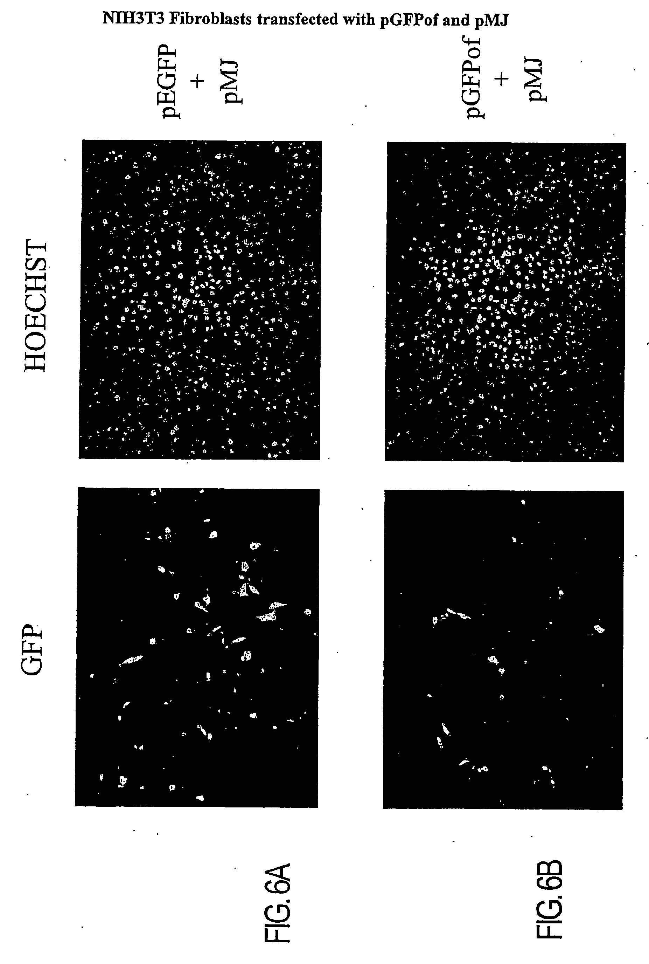

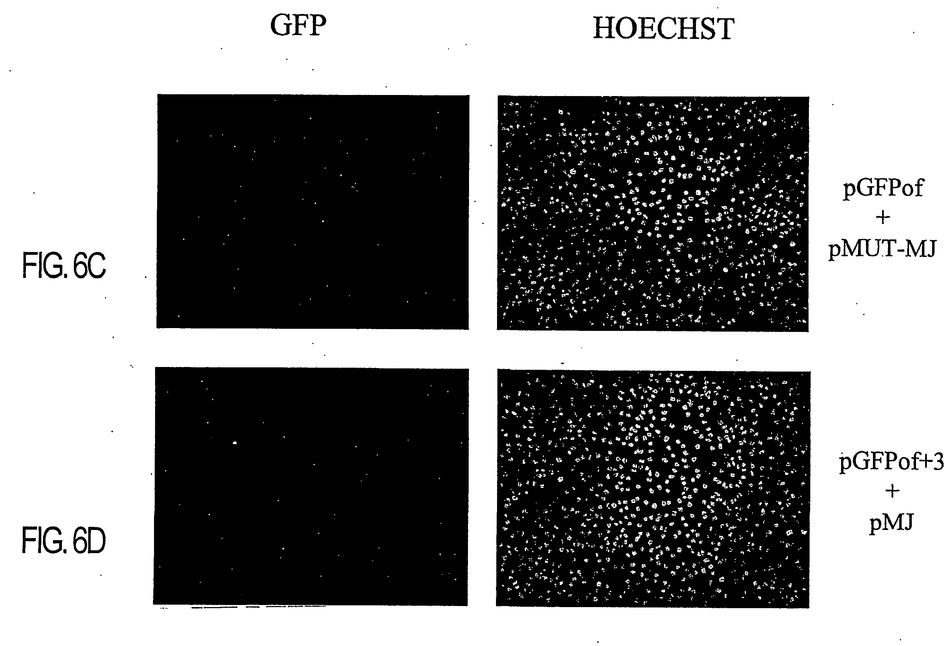

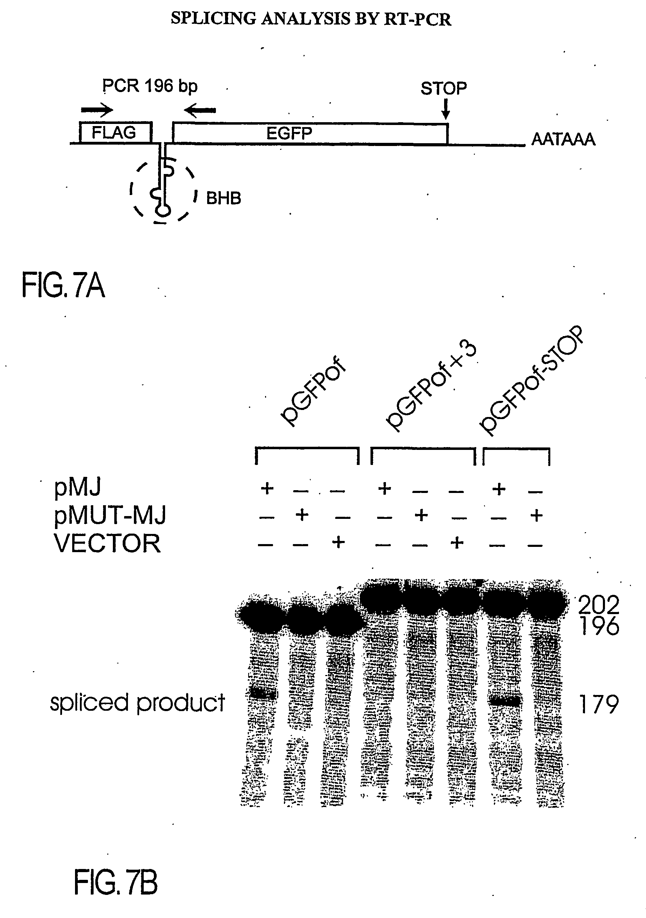

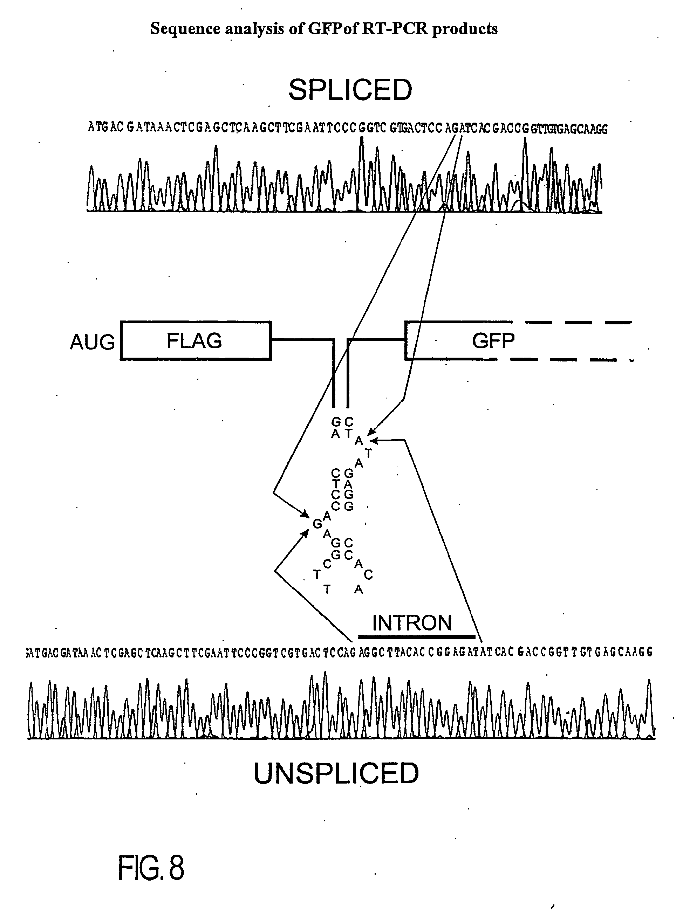

Fig. 6 shows that if 3T3 cells are transiently cotransfected with a plasmid expressing GFPof and a plasmid expressing the *M. jannaschii* endonuclease, GFP is produced. If a plasmid expressing an inactive endonuclease is utilized, GFP is not produced. These results suggest that the *M. jannaschii* endonuclease excises the intron and that an endogenous ligase activity produces a spliced mRNA that is correctly translated. This conclusion is substantiated by the finding that a new RNA species appears (Fig. 7) and that its sequence (Fig. 8) corresponds to that expected for spliced GFP mRNA. In addition, Fig. 7 shows that GFPof + 3 mRNA, a transcript characterized by an insertion of three base pairs in the helix of the BHB (Fig. 5), is not spliced. TRNA endonucleases do not tolerate the expansion of the helix of the BHB.

GFPof stop mRNA (Fig. 5), a transcript characterized by a twenty-three base long intron containing three stop codons in the three reading frames, is accurately spliced (Fig. 7).

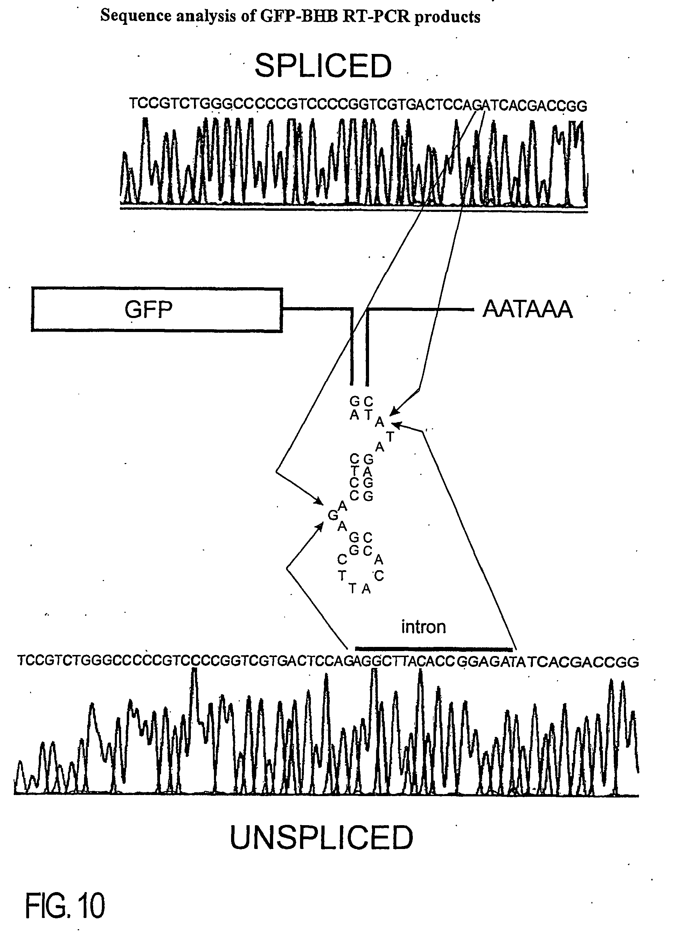

A spliced species is also produced when the BHB is not positioned in the coding region. In GFP-BHB mRNA (Fig. 5), the BHB is located in the 3' untranslated region. Fig. 9 shows that if 3T3 cells are transiently cotransfected with a plasmid expressing GFP-BHB and a plasmid expressing the *M. jannaschii* endonuclease, a new RNA species appears whose sequence (Fig. 10) corresponds to that of accurately spliced GFP mRNA.

### Structure-specific non-spliceosomal splicing of mRNA

The *Methanococcus Jannaschii* tRNA endonuclease recognizes and cleaves its normal substrates at bulge-helix-bulge (BHB) motifs that define the intron-exon boundaries in archaeal tRNAs and rRNAs. We show that this enzyme also cleaves on either end of a small intron inserted into a eukaryal mRNA encoding the green fluorescent protein (GFP). Moreover, the cleavage products are subsequently ligated together by endogenous RNA ligases in a reaction resembling that described for the HAC1 mRNA.

Introns included in the BHB, located in either the coding region or the 3' UTR, were excised from GFP mRNAs when the gene encoding the *M.Janaschii* enzyme was transfected into NIH3T3 cells. Our assays for proper ligation included detection of GFP by fluorescence microscopy and RT-PCR. Disruption of the BHB by extension of the helix results in no splicing.

These experiments demonstrate that the archaeal enzyme can recognize the BHB motif even in the context of a mammalian mRNA. This enzymatic system offers an opportunity to modulate gene expression *in vivo.*

### Materials and Methods

### Gene expression constructs

*Plasmids coding for mRNAs containing the BHB structure.*

### Splicing-dependent translation

GFP-OF was obtained by amplifying the GFP gene from the pEGFP-N3 expression vector (Clontech). The GFP was sequentially amplified with two set of primers: P1 VS P3 and P2 VS P3. A new start codon embedded in a Kozak sequence was inserted and the natural GFP start codon was mutated with the introduction an Agel site. Moreover, a FLAG epitope coding sequence separated from GFP was separated by the BHB structure. The FLAG and the GFP are coded on two different frames but they switch to the same frame when the BHB is correctly spliced. The modified GFP (GFP-OF) was re-inserted into the BgIII-NotI sites of the pEGFP-N3 vector (pGFP-OF).

Plasmids pGFP-OF+3 and pGFP-Stop were obtained by substitution of the *Eco*RI-*Age*I fragment of pGFP-OF with the double stranded oligonucleotides P4 and P5, respectively.

### Splicing-independent translation

Two different constructs containing the BHB in the 3' UTR were prepared, pGFP-BHB and pGFP-BHB+3. The GFP gene from the pd2EGFP-N1 vector (Clontech) was amplified with the primer pair P2 Vs P8 to add a 35-nucleotide long spacer downstream of the stop codon. The P2-P8 PCR product was then submitted to a new round of PCR with the P2-P6 and P2-P7 primer pairs in order to add the structures BHB and BHB+3, at the 3' end of GFP-BHB and GFP-BHB+3 products. These PCR products were digested with *Bgl*II and *Not*I and cloned back in the original vector.

### Plasmids coding for Methanococcucs Jannaschii tRNA endonuclease.

Construction pMJ plasmid. The *M. Jannaschii* gene with the FLAG epitope at its 5' end was cloned by PCR from the pET11 a bacterial expression vector (Hong Li, et al., Science, 280:284,1998) a kind gift of Dr. Christopher Trotta. The MJ-endoribonuclease PCR product was obtained using the primer pair P14 Vs P15 and cloned *Bgl*II*-Not*I in the pd2EGFP-N1 vector after excision of GFP.

The plasmid pMut-MJ was obtained via PCR from pMJ using mutagenic primers. Three aminoacidic substitutions were introduced: His 125 to PHE, LYS 156 to PRO and LYS157 to GLN. The *M. jannaschii* gene was amplified using three different primer pairs: P14 Vs P17, P16 Vs P19 and P18 Vs P5. All PCR products were mixed and re-amplified with P14 Vs P15. The mutagenized product was digested and cloned as was done for pMJ.

### Oligodeoxyribonucleotides

P1-GAGCTCAAGCTTCGAATTCCCGGTCGTGACTCCAGAGGCTTACACCGGAGATATCACGACCGGTTGTGAGCAAGGGCGAG
P2-ATCACGAGATCTCCACCATGGACTACAAAGACGATGACGATAAACTCGAGCTCAAGCTTCGAATT
P3-TCGGGATCCTCTACAAATGTGGTATGGCTG
P4-GCTTCGAATTCCCGGTCGTGACTTCTCCAGAGGCTTACACCGGAGAAGATATCACGACCGGTTGTGA
P5-GCTTCGAATTCCCGGTCGTGACTCCAGAGGTAACTGACTAAACCGGAGATATCACGACCGGTTGTGA
P6-ATAAGAATGCGGCCGCCCGGTCGTGATATCTCCGGTGTAAGCCTCTGGAGTCACGACCGGGGACGGG
P7-ATAAGAATGCGGCCGCCCGGTCGTGATATCTTCTCCGGTGTAAGCCTCTGGAGAAGTCACGACCGGGGACGGG
P8-TCACGACCGGGGACGGGGGCCCAGACGGAGGGCGAGTCCTTGTAGCGCATCTACACATTGATCCTAGCAGA
P9-CGTCAGATCCGCTAGCGCTAC
P10-CGTCGCCGTCCAGCTCGACCA
P11-TAGATGCGCTACAAGGACTCG
P12-TCGGGATCCTCTACAAATGTGGTATGGCTG
P14-ATCACGAGATCTCCACCATGGACTACAAAGACGATGACGATAAAATGGTGAGAGATAAAATG
P15-ATAAGAATGCGGCCGCGGATCCTTATGGTTTTACATAGG
P16-ATAAAGAATTCTCTGTTTATTTGGTTAAGG
P17-AACAGAGAATTCTTTATCATGTTAGCTCC
P18-TCAGTTCGGCCGCAATTACTCATAGCAATC
P19-GTAATTGCGGCCGAACTGAGTGAGCAACC
P20-GGCACCACCCCGGTGAACAG
P21-GTATGGCTGATTATGATCTAG

### Culture conditions and cell transfections

NIH3T3 fibroblasts were grown in Dulbecco's modified medium (DMEM) supplemented with 10% calf serum in a 37°C incubator with 5% CO₂. Cell transfections were carried out using the Lipofectamine 2000 or Lipofectamine Plus (Gibco-BRL) transfection reagent according to the manufacturer's instructions. Each transfection was performed in a 35 mm dish using 4 µg of total plasmid DNA with Lipofectamine 2000 or 2 µg with Lipofectamine Plus.

### RNA preparation and RT-PCR analysis

Total RNA was isolated from cells 24 hours after transfection using the Trizol reagent (Life Technologies) following the manufacturer's instructions. 10 µg of total RNA were treated with 1 unit RQ1 DNAse RNAse free(Promega M610A) for 30' at 37°C and phenol/chloroform extracted. Single-strand cDNA was obtained by polyT primed reverse transcription of 3 µg total RNA with SuperScript II RT (Life Technologies 18064-022).

pGFPOF,pGFPOF+ 3 and pGFPOF-STOP mRNAs splicing analysis was performed by PCR using the primer pair P9 Vs P10, whereas the primer pair P11 Vs P12 was used for pGFP-BHB and pGFP-BHB+3 analysis.

P9 or P12 primers were P³²labeled. PCR products were electrophoresed on a 6% polyacrylamide, 8M urea 20% formamide gel. The gels were dried and exposed to Phosphorimager for 16 hours and visualized by STORM 860 Phosphorimager (Molecular Dynamics).

### Sequencing of RT-PCR product

Spliced and unspliced amplified cDNAs were eluted from non-dried polyacrylamide denaturing gels and submitted to a new round of PCR. The P9-P10 primer pair was used for products derived from pGFPOF,pGFPOF+ 3 and pGFPOF-STOP mRNAs. P11-P12 was used for products derived from pGFP-BHB and pGFP-BHB+3. Sequence reactions were performed using a Bigdye sequencing kit (Perkin Elemer-Applied Biosystem) primed with P20 for products derived from the first plasmid group and with P21 for those derived from the second plasmid group. Sequences were analyzed on a ABI Prism 310 gentic analyser (Applied Biosystem).

### Immunoblotting analysis

Cell lysates were obtained with RIPA buffer (20 mM hepes PH 7.5, 1% Triton X-100, 150 mM NaCl, 10 mM EDTA), added with complete reagent (protease inhibitor cocktail tablets, Roche cat. 1697-498) and then subjected to SDS-PAGE (15%). Proteins were transferred to a PVDF membrane (immobilon P,Millipore). Membranes were blocked with 5% low-fat milk in TBST and incubated first with mouse anti-FLAG M2 monoclonal antibody (SIGMA F-3165) in Tris Buffered Saline (10mM tris-Cl at pH 7.5,150 mM NaCl) containing 0.05% Tween20 and then with HRP-conjugated goat anti-mouse IgG-1(γ) (Caltag M32007). Immunoreactive bands were detected by ECL (Supersignal West Pico Chemiluminescent Substrate, Pierce).

### Immunofluorescence

Detection of *Methanococcus Jannaschii* tRNA endonuclease. Twenty-four hours after plasmid transfection, cells were fixed with 2% paraformaldehyde/0.1 % Triton X100 for 20 minutes, washed three times with PBS/1%BSA and incubated with mouse anti-FLAG M2 monoclonal antibody (SIGMA F-3165) in PBS 1% BSA. Cells were then washed and incubated with FITC-conjugated goat anti-mouse IgG-1(γ) (Caltag cat. M32101).

Visualization of GFP protein in transfected cells. Forty-eight hours after transfection, cells were fixed with 2% paraformaldehyde/0.1 % Triton X100 for 20 minutes, incubated for 10 minutes with 1 µg/ml Hoechst /PBS 1X solution and washed twice with PBS.

All coverslips were mounted with 80% Glycerol/PBS mounting media. The images were taken by fluorescence microscopy (Olympus AX 70) with a Nikon digital camera (Coolpix 990) and processed with Adobe Photoshop version 5.0.

### References

Abelson, J., "RNA processing and the intervening sequence problem," Annu. Rev. Biochem. 48:1035-1069 (1979).
Baldi, M.I., Mattoccia, E., Ciafrè, S., Gandini-Attardi, D. and Tocchini-Valentini, G.P., "Binding and cleavage of pre-tRNA by the Xenopus splicing endonuclease: two separate steps in the intron excision reaction," Cell 47:965-971 (1986).
Baldi, M.I., Mattoccia, E., Bufardeci, E., Fabbri, S. and Tocchini-Valentini, G.P., "Participation of the intron in the reaction catalyzed by the Xenopus tRNA splicing endonuclease," Science 255:1404-1408 (1992).
Behlen, L.S., Sampson, J.R., Di Renzo, A.B. and Uhlenbeck, O.C., "Lead-catalyzed cleavage of yeast tRNAPhe mutants," Biochemistry 29:2515-2523 (1990).
Bufardeci, E., Fabbri, S., Baldi, M.I., Mattoccia, E. and Tocchini-Valentini, G.P., "In vitro genetic analysis of the structural features of the pre-tRNA required for determination fo the 3' splice site in the intron excision reaction," EMBO J. 12:4697-4704 (1993).
Carrara, G., Calandra, P., Fuscoloni, P. and Tocchini-Valentini, G.P., "Two helices plus a linker: a small model substrate for eukaryotic Rnase P," Proc. Natl. Acad. Sci. USA 92:2627-2631 (1995).
Gandini-Attardi, D., Margarit, I. and Tocchini-Valentini, G.P., "Structural alteration in mutant precursors of the yeast tRNALeu3 gene which behave as defective substrates for a highly purified splicing endoribonuclease," EMBO J. 4:3289-3297 (1985).
Gandini-Attardi, D., Baldi, M.I., Mattoccia, E. and Tocchini-Valentini, G.P., Transfer RNA splicing endonuclease from Xenopus laevis," Methods Enzymology 181:510-517 (1989).
Haselbeck, R.C. and Greer, C.L., "Minimum intron requirements for tRNA splicing and nuclear transport in Xenopus oocytes," Biochemistry 32:8575-8581 (1993).
Kierzek, R., "Nonenzymatic hydrolysis of oligoribonucleotides," Nucleic Acids Res. 20:5079-5084 (1992).
Kim, S.H., Quickley, G.J., Suddath, F.L., Mc Pherson, A., Sneden, D., Kim, JJ., Weinzierl, J. and Rich, A., "Three-dimensional structure of yeast Phenylalanine transfer RNA: folding of the polynucleotide chain," Science 179:285-288 (1973).
Kleman-Leyer, K., Arbruster, D.A. and Daniels, C.J., "Characterization of the Haloferax volcanii tRNA intron endonuclease gene reveals a relationship between the archaeal and eucaryal tRNA intron processing systems," Cell (accompanying paper).
Lee, M.C. and Knapp, G., "Transfer RNA splicing in Saccharomyces cerevisiae. Secondary and tertiary structures of the substrates," J. Biol. Chem. 260:3108-3115 (1985).
Mattoccia, E., Baldi, M.I., Gandini-Attardi, D., Ciafrè, S. and Tocchini-Valentini, G.P., "Site selection by the tRNA splicing endonuclease of Xenopus laevis," Cell 55:731-738 (1988).
Milligan, J.F. and Uhlenbeck, O.C., "Synthesis of small RNAs using T7 polymerase," Methods Enzymol. 180:51-62 (1989).
Miao, F. and Abelson, J., "Yeast tRNA-splicing endonuclease cleaves precursor tRNA in a random pathway," J. Biol. Chem. 268:672-677 (1993).
Nazarenko, I.A., Harrington, K.M. and Uhlenbeck, O.C., "Many of the conserved nucleotides of tRNAPhe are not essential for ternary complex formation and peptide elongation," EMBO J. 13:2464-2471 (1994).
Otsuka, A., De Paolis, A and Tocchini-Valentini, G.P., "Ribonuclease "XIaI", an activity from Xenopus laevis oocytes that excises intervening sequences from yeast transfer riboncleic acid precursors," Mol. Cell. Biol. 1:269-280 (1981).
Pan, T. and Uhlenbeck, O.C., "A small metalloribozyme with a two step mechanism," Nature 358:560-563 (1992).
Reyes, V.M. and Abelson, J., "A synthetic substrate for tRNA splicing," Anal. Biochem. 166:90-106 (1987).
Reyes, V.M. and Abelson, J., "Substrate recognition and splice site determination in yeast tRNA splicing," Cell 55:719-730 (1988).
Swerdlow, H. and Guthrie, C., "Structure of intron-containing tRNA precursors. Analysis of solution conformation using chemical and enzymatic probes," J. Biol. Chem. 259:5197-5207 (1984).
Tinoco, I., Jr., Borer, P.N., Dengler, B., Levine, M., Uhlenbeck, O.C., Crothers, D.M. and Gralla, J., "Improved estimation of secondary structure in RNAs," Nature New Biol. 246:40-41 (1973).
Trotta, C.R., Miao, F., Am, E.A., Stevens, S.W., Ho, C.K., Rauhut, R. and Abelson, J.N., "The yeast tRNA splicing endonuclease: a tetrameric enzyme with two active site subunits homologous to the Archeal tRNA splicing endonculeases, "Cell (accompanying paper).
Tuerk, C. and Gold, L., "Systematic evolution of ligands by Exponential enrichment: RNA ligands to bacteriophage T4 DNA polymerase," Science 249:505-510 (1990).
Westaway, S.K. and Abelson, J., "Splicing of tRNA Precursors. In tRNA: Structure, Biosynthesis, and Function," D. Soll and U.L. RajBhandary, eds (Washington, D.C.: ASM Press), pp. 79-92 (1995).
Wrede, P., Wurst, R., Vournakis, J. and Rich, A., "Conformational changes of yeast TRNAPhe and E. coli tRNAGlu as indicated by different nuclease digestion patterns," J. Biol. Chem. 254:9608-9616 (1979).

## Claims

1. An *in vitro* method of cleaving a target RNA molecule comprising the steps of:
exposing the target molecule in a cell to heterologous archaeal tRNA splicing endonuclease, wherein the target molecule is in a bulge-helix-bulge conformation created by two mRNA molecules, wherein the two mRNA molecules are the target RNA molecule and a second RNA molecule, and cleavage occurs between second and third nucleotides at bulges in the bulge-helix-bulge conformation and cleavage products are generated, wherein the target molecule does not comprise a tRNA structure, and
ligating the cleavage products from the target RNA and the second RNA so that a fusion RNA is formed comprising at least one cleavage product from the first target RNA molecule and at least one cleavage product from the second target RNA molecule.

2. The method of claim 1, wherein the cell is selected from the group consisting of mammalian, plant and eubacteria.

3. The method of claim 1, wherein the cell is a eukaryotic cell.

4. The method of claim 3, wherein the cell is mammalian.

## Patentansprüche

1. In vitro-Verfahren zum Spalten eines Ziel-RNA-Moleküls, das die Schritte umfasst:
Aussetzen des Zielmoleküls in einer Zelle gegenüber einer heterologen, archaealen tRNA-spleißenden Endonuklease, wobei das Zielmolekül in einer Bulge (Ausbauchung)-Helix-Bulge-Konformation vorliegt, die durch zwei mRNA-Moleküle erzeugt worden ist, wobei die zwei mRNA-Moleküle das Ziel-RNA-Molekül und ein zweites RNA-Molekül sind und die Spaltung zwischen dem zweiten und dem dritten Nukleotid an Ausbauchungen in der Bulge-Helix-Bulge-Konformation stattfindet und Spaltungsprodukte erzeugt werden, wobei das Zielmolekül keine tRNA-Struktur umfasst, und
Ligieren der Spaltungsprodukte von der Ziel-RNA und der zweiten RNA, so dass eine Fusions-RNA gebildet wird, die mindestens ein Spaltungsprodukt von dem ersten Ziel-RNA-Molekül und mindestens ein Spaltungsprodukt von dem zweiten Ziel-RNA-Molekül umfasst.

2. Verfahren nach Anspruch 1, bei dem die Zelle aus der Gruppe, bestehend aus einer Säuger-, einer Pflanzen- und einer Eubakterienzelle, ausgewählt ist.

3. Verfahren nach Anspruch 1, bei dem die Zelle eine eukaryotische Zelle ist.

4. Verfahren nach Anspruch 3, bei dem die Zelle eine Säugerzelle ist.

## Revendications

1. Procédé *in vitro* de clivage d'une molécule d'ARN cible, comprenant les étapes suivantes :
- exposer la molécule cible, dans une cellule, à l'action d'une endonucléase hétérologue archéenne d'épissage d'ARNt, laquelle molécule cible se trouve en une conformation renflement-hélice-renflement engendrée par deux molécules d'ARNm, ces deux molécules d'ARNm étant la molécule d'ARN cible et une deuxième molécule d'ARN, grâce à quoi un clivage se produit entre les deuxième et troisième nucléotides au niveau des renflements de la conformation renflement-hélice-renflement et il se forme des produits de clivage, et laquelle molécule cible ne comprend pas de structure d'ARNt,
- et ligaturer les produits de clivage issus de l'ARN cible et du deuxième ARN de manière à ce qu'il se forme un ARN de fusion comprenant au moins un produit de clivage issu de la première molécule d'ARN cible et au moins un produit de clivage issu de la deuxième molécule d'ARN cible.

2. Procédé conforme à la revendication 1, dans lequel la cellule est choisie parmi les cellules de mammifère, les cellules végétales et les eubactéries.

3. Procédé conforme à la revendication 1, dans lequel la cellule est une cellule eucaryote.

4. Procédé conforme à la revendication 3, dans lequel la cellule est une cellule de mammifère.
